# EUROPEAN PATENT SPECIFICATION

(11) **EP 0 430 325 B1**
(45) Date of publication and mention of the grant of the patent: **18.01.1995**
(21) Application number: 90202994.1
(22) Date of filing: 13.11.1990
(51) Int. Cl.: A01N 33/04, A01N 39/02, A01N 39/04, C07C 209/00

(54) **Process for the preparation of alkylamine salts**
Verfahren zur Herstellung von Alkylaminsalzen
Procédé de préparation de sels d'alkylamine

(30) Priority: 17.11.1989 NL 8902842
(43) Date of publication of application: 05.06.1991
(73) Proprietor: Akzo Nobel N.V., 6824 BM Arnhem (NL)
(72) Inventor: de Graaf, Johannes, NL-7548 AB Boekelo (NL)
(74) Representative: Schalkwijk, Pieter Cornelis

(56) References cited:
- EP-A- 0 062 161
- CHEMIKER-ZEITUNG, vol. 113, no. 9, September 1989, pages 261-271, Huthig VerlagGmbH, Heidelberg, DE; W. SCHROTH et al.: "Der Dimethylamin-Kohlendioxid-KomplexDimcarb und seine präparative Verwendung"

## Description

The invention relates to a process for the preparation of a salt from a primary or secondary alkylamine of which each alkyl group contains one to three carbon atoms and an acid.

It is known from the article by W. Schroth et al., "Der Dimethylamin-Kohlendioxid-Komplex Dimcarb und seine präparative Verwendung," Chemiker Zeitung, Vol. 113, No. 9, pp. 261-271, that use may be made of the anhydrous complex of dimethylamine.CO₂ in the reaction with alkyl halides, acid derivatives, active unsaturated aromatic halides, and enolizable carbonyl compounds. The article describes the process by which the complex is prepared from gaseous dimethylamine and powdered dry ice, the presence of any water being disadvised.
If no water is present, reacting the dimethylamine.CO₂ complex with an acid leads to amides being formed.
With respect to handleability extra precautions are required for such preparation and application of the anhydrous dimethylamine.CO₂ complex on account of the unfavourable vapour pressure, flash point, and boiling point of the anhydrous product. Furthermore, the non-dissolved product is difficult to feed and thus less suited to be used commercially.
However, it has now been found that preparing such an alkylamine.CO₂ complex in an aqueous solution is very simple and will give a readily handleable product without any extra precautions, while application of the product in solution in the preparation of herbicide salts will result in particular advantages.

The invention is characterized in that in a process according to the opening paragraph,
(a) CO₂ gas is being passed through an aqueous solution comprising the alkylamine and
(b) the solution is used to neutralize a herbicide with an acidic functional group.

Herbicides having an acidic functional group may be prepared in a conventional manner. Thus, for instance, herbicides derived from a phenoxy derivative are prepared by coupling an α-halide alkane carboxylic acid and a phenol derivative in an alkaline medium (pH in the range of about 9 to 12). Following acidification the free acid is obtained in the form of crystals that are poorly soluble in water.

In actual practice, therefore, herbicides having an acidic group are generally neutralized with an aqueous solution of alkylamine to make them soluble. However, the use of such amines will give odour nuisance, and the low flash point of the alkylamines makes it necessary to take strict and expensive precautions for the storage and transport of these materials.

It has been found that herbicides will go into solution faster if use is made of an aqueous alkylamine.CO₂ complex solution. Such solutions contain 15-70 wt.%, preferably 30-65 wt.%, and more particularly 40-60 wt.% of alkylamine in the form of a CO₂ complex.

The present invention offers a significant advantage in terms of the handleability of the formed substances. Herbicides, when neutralized in a conventional manner, have a pH in the range of 8-9. Such a high pH will give rise to irritation of the skin. Unlike in the case of unconverted alkylamine solutions being used in the reaction, the presently found process permits operation at a relatively low pH without crystallization of the formed herbicide salts. The pH of the neutralized solution may be in the range of 6-7,5, preferably of 6,5-7.

Further it was found that hardly any carcinogenic substabces are formed when storing an aqueous solution of an alkylamine CO2 complex. For example, it has been found the formation of N-nitrosodimethylamine, a carcinogenic substance formed when dimethylamine solutions are stored, is reduced significantly when the dimethylamine is converted with carbon dioxide prior to being stored. Storage of herbicides prepared according to the invention is also attended with a reduced formation of this carcinogenic substance, which is an additional advantage of the invention.

According to the present process it is preferred that the alkylamine.CO₂ complex be discontinuously added to the acid in a aqueous medium to be neutralized.
In this process the alkylamine.CO₂ complex will react with the acid to form the desired salt and CO₂, which will escape in the gas form.
Preferably, the herbicide acids are reacted with a complex formed by passing CO₂ gas through an aqueous alkylamine solution. More preferably, they are neutralized using an aqueous solution of alkylamines which prior to their addition to the herbicide acids were converted with a slight excess of carbon dioxide. By a slight excess is meant up to 7%, more specifically up to 5%. Though a higher volume can be passed through, it has been found that no benefit arises from doing so. In fact, it has been found that even an excess of 1 to 2% of CO₂ will suffice to reduce the amine odour of the finally formed salts to a very great extent.
Very favourable results are obtained when the herbicide acids are reacted with an aqueous solution of a dimethylamine.CO₂ complex which has been prepared by passing CO₂ gas through an aqueous dimethylamine solution.

Examples of herbicides having an acidic functional group include phenoxy acids, such as 2,4-dichlorophenoxy acetic acid,
2,4,5-trichlorophenoxy acetic acid,
2-(2-methyl-4-chlorophenoxy)propionic acid, and
2-methyl-4-chlorophenoxy acetic acid; 2-methoxy-3,6-dichlorobenzoic acid and 2,3,6-trichlorobenzoic acid, 4,6-dinitro-o-cresol and 2(sec butyl) 4,6-dinitrophenol.
Notably the reaction of phenoxy derivatives proceeds very satisfactorily, more particularly when 2-methyl-4-chlorophenoxy acetic acid or 2-(2-methyl-4-chlorophenoxy)propionic acid is used.

Herbicide acids often are foaming substances. Given that the production of foam is further reinforced by the escape of CO₂ gas during the salt formation, it is recommended to apply an anti-foaming agent in the conventional manner.

The alkylamines employed in the invention can be prepared in the usual manner, e.g. by an alkanol or alkyl halide being reacted with ammonia and the subsequent isolation of the alkylamine formed. Such alkylamines, which have alkyl groups containing 1 to 3 carbon atoms, are readily soluble in water. Examples of such alkylamines include mono-and dimethylamine, mono- and diethylamine, mono- and di-isopropylamine, and mono-and di-n-propylamine. Alternatively, mixtures of these amines may be used. Preference is given to the use of dimethylamine.

Commonly, the lower alkylamines are dissolved for transporting, although monomethylamine is transported in the gas form as well. Strict safety precautions are required for the transport of alkylamine solutions on account of the fire and explosion hazards. This is also the case for the anhydrous dimethylamine.CO₂ complex. It has now been found that, if alkylamines are contacted with CO₃²⁻ in an aqueous solution, the resulting aqueous complex containing solution has a significantly higher flash point and, as a result, is no longer explosive.

Preference is therefore given to the alkylamines being converted in an aqueous solution with the compounds formed by passing CO2 trough an aquous solution prior to their being transported to the place where the alkylamine salt according to the invention is prepared. An additional advantage here is that the formation of the aforementioned carcinogenic compound during transport is reduced. The preferred means of converting alkylamines in the CO₂ complexes is CO₂ gas being passed through or formed in an aqueous solution of the alkylamines. Although using carbonate salts or solutions thereof may also lead to the desired conversion, preference is given to conversion with CO₂ gas, since carbonate salts will introduce undesired cations.

According to the process now found at least 90% of the amine present is converted with CO₂, and preferably at least 93%. Optimum results are attained with full conversion.
If the complex is formed by CO₂ gas being passed through an aqueous alkylamine solution, the rate at which the gas is contacted with the alkylamines is not critical. In actual practice, the skilled man will pass so much CO₂ gas through an alkylamine solution as will permit the reaction temperature being kept at a constant level with the use of the available cooling equipment. In general, the reaction temperature will be in the range of -20° to 60°C, preferably in the range of 10° to 50°C, and more particularly in the range of 15° to 35°C.

The invention will be illustrated with reference to the following examples.

### Examples

### Example A

In a reaction vessel for 5 hours CO₂ gas was passed at a rate of 40 l/min through 60 kg of a 60%-dimethylamine solution having a vapour pressure of about 40 kPa at 20°C and a flash point of -40°C. The viscosity of the solution was 2 mPa.s. The reaction temperature was kept at 25°C. The total amount of converted dimethylamine solution formed was 79 kg. The reaction product formed was virtually odourless. The DMA vapour pressure at 20°C was about 1-2 kPa. Because of a flash point of 34°C the reaction product was considerably safer to transport and treat. The pH of the solution had decreased from over 13 to about 10. The viscosity of the solution was 46 mPa.s

### Example B

In the same manner as described in Example A CO₂ was passed through a 40%-dimethylamine solution having a flash point of -19°C in a stoichiometric ratio. The solution formed was practically odourless, its flash point about 38°C.

### Example C

A 40%-monomethylamine solution (flash point -10°C) was contacted at 15°C with a stoichiometric amount of CO₂ gas. The resulting virtually odourless solution had a flash point of 35°C.

### Example D

The reaction product formed in Example A was analysed after four weeks' storage.

It was found that 5 ppm of nitroso-dimethylamine had formed. Analysis of an untreated sample of a 60%-dimethylamine solution showed that, under the same conditions, after 4 weeks 22 ppm of nitroso-dimethylamine had formed.

### Example I

To 235 g of water in a reaction vessel were added 70 g of 2-(2-methyl-4-chlorophenoxy)propionic acid (MCPP) and a small amount of an anti-foaming agent (silane). The temperature was raised to 50°C, whereupon 315 g of the solution obtained in Example B were added at a rate of 8 g/min. In the meantime, so much MCPP was added as would ensure that some undissolved MCPP was continually present in the reaction medium. During the reaction a thin layer of foam was formed. Shortly after the last addition the escape of CO₂ came to a virtual halt. No heat was generated during the addition of the 2-dimethylamine.CO₂ solution.
The pH of the solution had increased to 6,8.

### Example II

Introduced into a reaction vessel were 176 g of water containing some anti-foaming agent, after which about 10% of the total amount of 2-methyl-4-chlorophenoxy acetic acid (MCPA) to be fed was added. Next, 432 g of the solution obtained in Example B were added dropwise, with simultaneous feeding of so much MCPA as would ensure that some undissolved MCPA was continually present in the vessel. In all, 635 g of MCPA were added.
The final product had a pH of 7.

### Example III

In the same manner as described in Example I 630 g of 2,4-dichlorophenoxy acetic acid in 275 g of water were reacted with 386 g of the solution obtained in Example B. The pH of the resulting solution was 6,5.

In the Examples I, II, and III a phenol-like odour, originating from the herbicide, was detected up to the last amine addition inclusive. Vigorous stirring during the reaction in order to dissolve the solid acid particles was no longer found to be necessary, since they already dissolved under the influence of gas generation.

### Example IV

1000 kg of a 2.dimethylamine.CO₂ solution were prepared as described in Example A, introduced into vessels of polyethylene, and transported to a herbicide manufacturing plant. The polyethylene vessels were transported on an open platform under the transport category of substances with a flash point between 21° and 55°C. No additional precautions with respect to inhalation were required, so that the transport could take place in significantly simpler fashion than had always been customary for dimethylamine.

Charged into a 4 m³ reactor were 1269 kg of water to which 125 kg of 2-methyl-4-chlorophenoxy acetic acid (MCPA) had been added. Next, the transported 2.dimethylamine.CO₂ solution and MCPA were added stepwise until 1550 kg of MCPA and 755 kg of amine solution in all had been introduced into the reaction vessel. During the reaction an anti-foaming agent was added in an amount of 0,1% by weight, calculated on the total amount of solution, after which hardly any foam was formed anymore.

The reaction temperature was kept at 40°C. The neutralization reaction proceeded very rapidly, so that all the herbicide had dissolved virtually directly after the last amine addition. The pH was 6,8.
The resulting clear final product did not have a dimethylamine odour but a cresol-like odour, which originated from the herbicide.
A hard water test was carried out, to which end the herbicide.dimethylamine was diluted to 4% with synthetic hard water. This synthetic hard water contained 0,04 M of CaCO₃ and 0,004 M of MgO.
To determine the cold stability the herbicide.dimethylamine was placed in a refrigerator (T = 5°C) overnight. No crystallization was observed either in the hard water test or in the test for determining the cold stability.
This in contradistinction to herbicide neutralized with non-converted dimethylamine, which at the same pH does crystallize.

## Claims

1. A process for the preparation of a salt from a primary or secondary alkyl amine of which each alkyl group contains one to three carbon atoms and an acid, characterised in that
(a) CO₂ gas is being passed through an aqueous solution comprising the alkyl amine and
(b) the solution so obtained is used to neutralize a herbicide with an acidic functional group.

2. A process according to claim 1, characterized in that the alkylamine is reacted with CO₂ in an aqueous solution prior to being transported to the place where the herbicide salt is prepared.

3. A process according to any one of the preceding claims, characterized in that the alkyl amine used is a dimethylamine.

4. A process according to any one of the preceding claims, characterized in that the herbicide having an acidic functional group is a phenoxy acid derivative.

5. A process according to claim 4, characterized in that the herbicide having an acidic functional group is 2-methyl-4-chlorophenoxy acetic acid.

6. A process according to claim 4, characterized in that the herbicide having an acidic functional group is 2-(2-methyl-4-chlorophenoxy)propionic acid.

7. A process according to any one of the preceding claims, characterized in that the reaction is carried out in the presence of an anti-foaming agent.

## Patentansprüche

1. Verfahren zur Herstellung eines Salzes aus einem primären oder sekundären Alkylamin, dessen Alkylgruppen je 1 bis 3 Kohlenstoffatome enthalten, und einer Säure, dadurch gekennzeichnet, dass
(a) CO₂-Gas durch eine wässrige Lösung geleitet wird, die das Alkylamin enthält, und
(b) die so erhaltene Lösung zur Neutralisierung eines Herbicids mit einer sauren funktionellen Gruppe verwendet wird.

2. Verfahren nach Anspruch 1, dadurch gekennzeichnet, dass das Alkylamin vor dem Transport zum Ort der Herstellung des Herbicidsalzes mit CO₂ in wässriger Lösung umgesetzt wird.

3. Verfahren nach einem der vorangehenden Ansprüche, dadurch gekennzeichnet, dass das verwendete Alkylamin Dimethylamin ist.

4. Verfahren nach einem der vorangehenden Ansprüche, dadurch gekennzeichnet, dass das Herbicid mit saurer funktioneller Gruppe ein Phenoxysäurederivat ist.

5. Verfahren nach Anspruch 4, dadurch gekennzeichnet, dass das Herbicid mit saurer funktioneller Gruppe 2-Methyl-4-chlorphenoxyessigsäure ist.

6. Verfahren nach Anspruch 4, dadurch gekennzeichnet, dass das Herbicid mit saurer funktioneller Gruppe 2-(2-Methyl-4-chlorphenoxy)propionsäure ist.

7. Verfahren nach einem der vorangehenden Ansprüche, dadurch gekennzeichnet, dass die Umsetzung in Gegenwart eines Antischaummittels durchgeführt wird.

## Revendications

1. Un procédé de préparation d'un sel à partir d'une alkylamine primaire ou secondaire dont chaque groupe alkyle contient un à trois atomes de carbone, et d'un acide, caractérisé en ce que :
(a) du CO₂ gazeux est envoyé à travers une solution aqueuse comprenant l'alkylamine et
(b) la solution ainsi obtenue est utilisée pour neutraliser un herbicide ayant un groupe fonctionnel acide.

2. Un procédé suivent la revendication 1, caractérisé en ce que l'alkylamine réagit avec du CO₂ dans une solution aqueuse avant d'être transportée sur le lieu où le sel d'herbicide est préparé.

3. Un procédé suivant l'une quelconque des revendications précédentes, caractérisé en ce que l'alkylamine utilisée est une diméthylamine.

4. Un procédé suivant l'une quelconque des revendications précédentes, caractérisé en ce que l'herbicide ayant un groupe fonctionnel acide est un dérivé de phénoxy acide.

5. Un procédé suivant la revendication 4, caractérisé en ce que l'herbicide ayant un groupe fonctionnel acide est l'acide 2-méthyl-4-chlorophénoxy acétique.

6. Un procédé suivant la revendication 4, caractérisé en ce que l'herbicide ayant un groupe fonctionnel acide est l'acide 2-(2-méthyl-4-chlorophénoxy)propionique.

7. Un procédé suivant l'une quelconque des revendications précédentes, caractérisé en ce que la réaction est conduite en présence d'un agent anti-mousse.
